(19) **Europäisches Patentamt**

**European Patent Office**

**Office européen des brevets**

(11) **EP 0 906 569 B1**

(12) **EUROPÄISCHE PATENTSCHRIFT**

(45) Veröffentlichungstag und Bekanntmachung des Hinweises auf die Patenterteilung:
**14.11.2001 Patentblatt 2001/46**

(51) Int Cl.[7]: **G01N 33/52**, G01N 33/72, G01N 33/68, G01N 33/84

(21) Anmeldenummer: 97925031.3

(22) Anmeldetag: **30.05.1997**

(86) Internationale Anmeldenummer:
**PCT/EP97/02834**

(87) Internationale Veröffentlichungsnummer:
**WO 97/45732 (04.12.1997 Gazette 1997/52)**

(54) **VERFAHREN ZUR ANALYSE HÄMOGLOBIN ENTHALTENDER MEDIZINISCHER PROBEN**

PROCESS FOR THE ANALYSIS OF MEDICAL SAMPLES CONTAINING HAEMOGLOBIN

PROCEDE D'ANALYSE D'ECHANTILLONS MEDICAUX CONTENANT DE L'HEMOGLOBINE

(84) Benannte Vertragsstaaten:
**AT BE CH DE DK ES FI FR GB IE IT LI NL PT SE**

(30) Priorität: **31.05.1996 DE 19622089**

(43) Veröffentlichungstag der Anmeldung:
**07.04.1999 Patentblatt 1999/14**

(73) Patentinhaber: **Roche Diagnostics GmbH**
**68305 Mannheim (DE)**

(72) Erfinder:
• **WEISHEIT, Ralph**
  **D-82362 Weilheim (DE)**
• **SCHELLONG, Lieselotte**
  **D-82327 Tutzing (DE)**

(74) Vertreter: **Weiss, Wolfgang, Dipl.-Chem. Dr. et al**
**Weickmann & Weickmann**
**Patentanwälte**
**Postfach 86 08 20**
**81635 München (DE)**

(56) Entgegenhaltungen:
**DE-A- 4 427 492**

• **O. SONNTAG: "Haemolysis as an interference factor in clinical chemistry" JOURNAL OF CLINICAL CHEMISTRY AND CLINICAL BIOCHEMISTRY, Bd. 24, Nr. 2, 1986, NEW YORK NY USA, Seiten 127-139, XP002042642 in der Anmeldung erwähnt**
• **B. HAHN ET AL.: "Polychromatic analysis: new applications of an old technique." CLINICAL CHEMISTRY, Bd. 25, Nr. 6, 1979, WINSTON-SALEM SC USA, Seiten 951-959, XP002037050 in der Anmeldung erwähnt**

**Beschreibung**

Die Erfindung betrifft ein Verfahren zur Bestimmung eines Analyten in einer freies Hämoglobin enthaltenden Probe, wobei die Bestimmung durch eine optische Messung erfolgt und der für die Analytkonzentration gemessene Wert rechnerisch korrigiert wird. Insbesondere ist dieses Verfahren zur Bestimmung der Parameter Gesamtprotein, Eisen und Albumin in einer medizinischen Probe, z. B. in einer Serum- oder Plasmaprobe geeignet.

Es ist allgemein bekannt, daß durch Hämolyse die Bestimmung einer Vielzahl von Analyten in teilweise erheblichem Maße gestört ist. Um dennoch unverfälschte Meßwerte zu erhalten, wurden in der Vergangenheit unterschiedliche Verfahren zu Hämolyse-Entstörung publiziert.

Wie im Patent EP-0 268 025 B1 erwähnt, wurde für einzelne Analyten ein graphischer Zusammenhang hergestellt zwischen Hämolysegrad und resultierendem Meßfehler. Daraus konnten Korrekturfaktoren abgeleitet werden, mit welchen dann auf Basis einer separaten Bestimmung des Hämolysegrades das erhaltene Analysenergebnis rechnerisch korrigiert wurde.

Jay und Provasek beschrieben ebenfalls, daß durch Bestimmung des Hämolysegrades und Verwendung eines Korrekturfaktors unverfälschte Werte in hämolytischen Proben erhalten werden können (Clin Chem 38/6, 1026 (1992) bzw. Clin Chem 39/9, 1804-1810 (1993)). Hier wird der Hämolysegrad durch eine separate Bestimmung des Hb-Gehalts in der Probe ermittelt.

In der Patentschrift US 4,263,512 wird empfohlen, neben dem Analyten den Grad Trübung (X), Hämolyse (Y) und Ikterus (Z) zu bestimmen und den gemessenen Analytwert (S) mit Hilfe der Formel $S' = S - \alpha \cdot X - \beta \cdot Y - \gamma \cdot Z$ zu korrigieren. Dabei sind S' der korrigierte Analytwert und $\alpha$, $\beta$ und $\gamma$ Korrekturfaktoren, die durch Messung des Einflusses von Trübung, Hämolyse und Ikterus mittels Referenzflüssigkeiten erhalten wurden. Die Ermittlung von X, Y und Z erfolgt durch Mehrkanalmessung und anschließender komplizierter Berechnung aus den erhaltenen Absorptionsdifferenzen unter Berücksichtigung des Anteils der jeweils anderen interferierenden Substanzen.

Ein Weg zur Korrektur von Hämolysestörungen ohne separate Bestimmung des Hämolysegrades wird durch DE 44 27 492 A1 aufgezeigt. Hier wurde ein mathematischer Zusammenhang gefunden zwischen dem Gehalt an durch Hämolyse aus den Erythrocyten freigesetzter Störsubstanz und einer vor der Hauptreaktion ablaufenden Vorreaktion. Mit Hilfe des somit während der Vorreaktion ermittelten Hämolysegrades kann das in der Hauptreaktion (Rate $_{total}$) erhaltene Analysenergebnis unter Ausnutzung des gefundenen Zusammenhangs zwischen Hämolysegrad und Störbeitrag entsprechend der Formel $Rate_{Substanz/Probe} = Rate_{total} - Rate_{Vorreaktion} - Rate_{Substanz/Erythrocyt}$ korrigiert werden, wobei Substanz die zu bestimmende Komponente in der Probe bedeutet.

Häufig ist eine Störung durch Hämoglobin auch durch Messung des Probenleerwerts zu eliminieren. Dies gilt jedoch nicht für die Bestimmung von Totalprotein mittels Biuret-Methode (Morgan et al. Microchem J 44, 282-287 (1991)) und auch nicht für die Bestimmung von Albumin mittels Bromcresolgrün- bzw. -purpur-Methode. Für die Bestimmung von Eisen ist weiterhin bekannt, daß es stets dann zu Störungen durch Hämoglobin kommt, wenn Hb nicht vorher durch Dialyse aus der Probe entfernt wurde (Sonntag, J Clin Chem Clin Biochem 24/2, 127-139 (1986)). Auch bei Eisen ist die Hb-Störung nicht durch alleinige Messung des Probenleerwerts zu beseitigen.

Alle oben beschriebenen Entstörungsmethoden haben jedoch den Nachteil, daß sie mit erheblichem Arbeitsaufwand (Probenvorbereitung durch Dialyse bzw. separate Ermittlung des Hämolysegrades z. B. durch Bestimmung des Hb-Gehalts) und/oder komplizierten mathematischen Korrekturalgorithmen verbunden sind.

Außerdem sind die beschriebenen Verfahren alle bezogen auf Entstörung von durch Hämolyse verursachten Fehlmessungen. Mit der Entwicklung von Blutersatzmitteln auf Basis von Hämoglobin stellt sich die Frage nach der Beseitigung von Störungen durch natives oder synthetisches Hämoglobin bzw. Hb-analogen Verbindungen noch weit brisanter als bisher. Solche Störungen treten dann zum einen auch in nichthämolytischem Probenmaterial und zum anderen auch in weit höherem Grad auf als bei nativer Hämolyse, da bei Therapie mit Blutersatzmitteln der Hb-Gehalt im Blutserum oder -plasma mehr als 1000 mg/dl betragen kann.

Aufgabe der vorliegenden Erfindung war es, ein Verfahren zur Beseitigung von Störungen bereitzustellen, die durch natives Hämoglobin oder auf synthetischem Hb bzw. Hb-analogen Verbindungen basierenden Blutersatzmitteln hervorgerufen werden und nicht durch einfache Messung des Probenleerwerts zu beseitigen sind. Dieses Verfahren sollte außerdem gegenüber herkömmlichen Verfahren mit signifikant reduziertem Arbeitsaufwand verbunden sein und eine Entstörung bis mindestens 1000 mg/dl Hb garantieren.

Gelöst wurde die der Erfindung zugrunde liegende Aufgabe dadurch, daß überraschenderweise ein Zusammenhang gefunden wurde zwischen der Höhe des Probenleerwerts und dem Grad der Verfälschung der Meßergebnisse durch freies Hämoglobin. Dadurch ist es möglich, mit Hilfe einer einfachen mathematischen Korrekturformel auch bei großem Hb-Gehalt den korrekten Wert für die Analytkonzentration genau zu ermitteln. Im Vergleich zum Stand der Technik zeichnet sich das erfindungsgemäße Verfahren vorteilhaft dadurch aus, daß weder eine separate Ermittlung des Hb-Gehalts noch eine Bestimmung des Grades einer Vorreaktion erforderlich sind, um den Meßwert einer Hb-haltigen medizinischen Probe zu korrigieren.

Ein Gegenstand der vorliegenden Erfindung ist somit ein Verfahren zur Bestimmung eines Analyten in einer freies Hämoglobin enthaltenden Probe durch optische Messung, wobei man den gemessenen Wert für die Analytkonzentration korrigiert durch die Schritte:

(a) Messen des Leerwerts der zu analysierenden Probe,

(b) Messen des Leerwerts einer Hämoglobin freien Referenzprobe,

(c) Messen des unkorrigierten Werts für die Analytkonzentration und

(d) Korrigieren des in Schritt (c) erhaltenen Werts durch Korrelation mit den in Schritt (a) und (b) erhaltenen Werten, um den korrigierten Wert für die Analytkonzentration zu erhalten.

Der korrigierte Wert für die Analytkonzentration in Schritt (d) des erfindungsgemäßen Verfahrens wird vorzugsweise nach folgender Beziehung ermittelt:

$$C'_{Probe} = C_{Probe} - F \cdot E1_{Probe} + F \cdot E1_{Referenz}$$

wobei

$C'_{Probe}$ der korrigierte Wert für die Analytkonzentration ist,

$C_{Probe}$ der unkorrigierte gemessene Wert für die Analytkonzentration in der Probe ist,

F ein testspezifischer Korrekturfaktor ist,

$E1_{Probe}$ der gemessene Leerwert in der Probe ist und

$E1_{Referenz}$ der gemessene Leerwert in der Referenzprobe ist.

Die erfindungsgemäße Korrekturmethode ist für Verfahren geeignet, bei denen die Bestimmung des Analyten durch optische Messung erfolgt, insbesondere durch optische Messung bei Wellenlängen, bei denen eine Störung durch freies in der Probe vorliegendes Hämoglobin eintritt. Besonders bevorzugt erfolgt die optische Messung im Bereich von 500-750 nm.

Das erfindungsgemäße Verfahren eignet sich zur Bestimmung beliebiger Proben, in denen freies Hämoglobin vorliegt. Beispiele für solche Proben sind hämolytische Serum- oder Plasmaproben oder Proben, die ein Blutersatzmittel enthalten. Beispiele für Blutersatzmittel, die im Sinne der vorliegenden Erfindung unter den Begriff "freies Hämoglobin" fallen, sind derivatisierte, polymerisierte, modifizierte oder quervernetzte Derivate von Hämoglobinen, insbesondere von Humanhämoglobin oder Rinderhämoglobin, sowie rekombinant hergestelltes Hämoglobin.

In einer bevorzugten Ausführungsform des erfindungsgemäßen Verfahrens wird der Gesamtproteingehalt der Probe bestimmt. Diese Bestimmung erfolgt vorzugsweise nach der Biuret-Methode. In einer weiteren besonders bevorzugten Ausführungsform der vorliegenden Erfindung wird der Eisengehalt der Probe bestimmt. Diese Bestimmung des Eisengehalts erfolgt vorzugsweise nach der Ferrozin-Methode. In noch einer weiteren besonders bevorzugten Ausführungsform wird der Albumingehalt einer Probe bestimmt. Diese Bestimmung des Albumingehalts erfolgt vorzugsweise nach der Bromcresolgrün- oder Bromcresolpurpur-Methode. Bei der Bestimmung dieser Parameter, bei denen bisher keine einfache Entstörungsmethode für die Bestimmung Hämoglobin enthaltender Proben bekannt war, wird durch das erfindungsgemäße Verfahren überraschenderweise eine deutliche Vereinfachung der Meßprozedur erreicht.

Weiterhin zeichnet sich das erfindungsgemäße Verfahren dadurch aus, daß selbst ikterische Proben mit einem hohen Bilirubingehalt bis mindestens 20 mg/dl problemlos vermessen werden können. Eine Störung durch lipämische Proben kann durch Verwendung eines entsprechenden Aufhellmittels, welches z. B. dem Reagenz zugesetzt ist, eliminiert werden.

Als Probe wird beim erfindungsgemäßen Verfahren vorzugsweise eine Serum- oder Plasmaprobe eingesetzt, insbesondere eine humane Serum- oder Plasmaprobe. Als Referenzproben werden günstigerweise Serum- oder Plasmaproben von klinisch gesunden Probanden verwenden. Besonders bevorzugt wird ein Hämoglobinfreier Serum- oder Plasmapool klinisch gesunder Probanden verwendet.

Ein besonderer Vorteil des erfindungsgemäßen Verfahrens besteht darin, daß es in einem Analyseautomaten durchgeführt werden kann, z. B. an einem Boehringer Mannheim/Hitachi 704 oder 717 Analysegerät. Aufgrund der einfachen mathematischen Korrekturformel kann der Analyseautomat so programmiert werden, daß bereits der korrigierte Wert für die Analytkonzentration ausgegeben wird und eine nachträgliche rechnerische Korrektur nicht mehr erforderlich ist.

Ein wesentlicher Parameter für die Korrektur der gemessenen Analytkonzentration ist der testspezifische Korrekturfaktor F. Dieser Korrekturfaktor F wird vorzugsweise durch ein Verfahren ermittelt, welches die Schritte umfaßt:

(a) Bereitstellen einer Serie von mindestens 3 Proben mit gleichem Analytgehalt, wobei mindestens eine der Proben kein Hämoglobin enthält und mindestens 2 der Proben unterschiedliche Konzentrationen an freiem Hämoglobin enthalten,

(b) Messen des Leerwerts von jeder Probe, wobei der durch Anwesenheit von Hämoglobin verursachte Anstieg des Probenleerwerts gegenüber der Hämoglobin-freien Probe bestimmt wird,

(c) Messen der unkorrigierten Analytkonzentration in jeder Probe, wobei die durch Anwesenheit von Hämoglobin verursachte Meßwertverfälschung gegenüber der Hämoglobin-freien Referenzprobe bestimmt wird, und

(d) Korrelation der in Schritt (c) bestimmten Meßwertverfälschung mit dem in Schritt (b) bestimmten Anstieg des Probenleerwerts, um den testspezifischen Korrekturfaktor zu erhalten.

Günstigerweise wird bei der Bestimmung des Korrekturfaktors F eine Serie von Proben bereitgestellt, wobei mindestens 5 und beispielsweise 10 der Proben unterschiedliche Konzentrationen an freiem Hämoglobin enthalten. Die Konzentrationen an freiem Hämoglobin werden für die Probenserie beispielsweise im Bereich von 0 mg/dl bis mindestens 1000 mg/dl variiert.

Für eine bestimmte, freies Hämoglobin enthaltende Probe aus der Serie wird ein probenspezifischer Korrekturfaktor F' nach folgender Beziehung ermittelt:

$$F' = \Delta C : \Delta E1$$

wobei:

$\Delta C$ der für jeweils eine Probe durch die Anwesenheit von freiem Hämoglobin verursachte Betrag der Meßwertverfälschung gegenüber der Referenzprobe ist ist und

$\Delta E1$ der für jeweils eine Probe durch die Anwesenheit von freiem Hämoglobin verursachte Anstieg des Probenleerwerts gegenüber der Referenzprobe ist.

Aus den für die jeweiligen Proben bestimmten Korrekturfaktoren F' kann der testspezifische Korrekturfaktor F durch Bildung des Mittelwerts ermittelt werden. Auf diese Weise konnte beim Nachweis von Albumin durch die Bromcresolgrün-Methode ein Korrekturfaktor F von 0,332, beim Nachweis von Eisen durch die Ferrozin-Methode ein Korrekturfaktor F von 0,290 und beim Nachweis von Gesamtprotein durch die Biuret-Methode ein Korrekturfaktor von 0,115 ermittelt werden. Bei Anwendung dieser Korrekturfaktoren wurde eine hervorragende Wiederfindungsrate des zu bestimmenden Analyten bei hämoglobinhaltigen Proben gefunden.

Weiterhin wird die Erfindung durch die nachfolgenden Beispiele erläutert:

Allgemeine Methoden

**1. Ermittlung des Korrekturfaktors (siehe auch Beispiele 1-3):**

Aus einem Serum- oder Plasmapool klinisch gesunder Probanden werden 11 Proben mit unterschiedlichen Mengen Hämolysat, Hämoglobin oder einer Hb-analogen Verbindungen derart aufgestockt, daß bei gleichbleibendem Analytgehalt eine Hb-Konzentrationsreihe entsteht, deren niedrigste Probe (= Referenz) kein Hb und deren höchste Probe mindestens 1000 mg/dl Hb enthält. Alle Proben dieser Reihe werden mit dem jeweiligen Test vermessen, wobei für jede Probe ein entsprechend ihres Hb-Gehalts gegenüber der Referenz verfälschter Analytwert erhalten wird.

Für jede Probe wird der durch Hb verursachte Anstieg des Probenleerwerts $\Delta E1$ gegenüber dem Probenleerwert der Hb-freien Probe (= Referenz) ermittelt: $\Delta E1 = E1_{Probe} - E1_{Referenz}$. Weiterhin wird für jede Probe der durch Hb verursachte Betrag der Analytenwertverfälschung $\Delta C$ gegenüber dem in der Referenz gemessenen Analytwert

ermittelt: $\Delta C = C_{Probe} - C_{Referenz}$.

Bei Division des Störanteils $\Delta C$ durch den Betrag des Probenleerwertanstiegs $\Delta E1$ erhält man für jede Probe einen Korrekturfaktor $F'_{Probe} = \Delta C : \Delta E1$.

Aus den somit erhaltenen 10 Einzelfaktoren der Hb-Konzentrationsreihe wird nun der gemittelte Korrekturfaktor F gebildet. Dieser Faktor ist eine feste Größe, der für Albumin, Eisen und Total Protein einmalig ermittelt werden muß, für den jeweiligen Test dann jedoch konstant ist.

**2. Berechnung des korrigierten Analytwerts (siehe auch Beispiele 4-8) :**

Durch rechnerische Korrektur des gemessenen Analytwerts der Probe $C_{Probe}$ um den Störanteil $\Delta C$ wird der korrigierte und damit ungestörte Analytwert der jeweiligen Probe $C'_{Probe}$ ermittelt:

$$C'_{Probe} = C_{Probe} - \Delta C$$

$$C'_{Probe} = C_{Probe} - F \cdot \Delta E$$

$$C'_{Probe} = C_{Probe} - F \cdot (E1_{Probe} - E1_{Referenz})$$

$$C'_{Probe} = C_{Probe} - F \cdot E1_{Probe} + F \cdot E1_{Referenz}.$$

Die Referenz ist, wie oben beschrieben, ein Hb-freier Serum- oder Plasmapool klinisch gesunder Probanden. Für die genannten Methoden ist der Einfluß der Schwankungsbreite von gemessenen Probenleerwerten verschiedener Patientenproben aufgrund des Verhältnisses von Probenleerwert zum Meßsignal vernachlässigbar gering. Selbst ikterische Proben mit einem Bilirubingehalt bis mindestens 20 mg/dl stören nicht. Eine Störung durch lipämische Proben kann durch Verwendung eines entsprechenden Aufhellmittels, welches z. B. dem Reagenz zugesetzt ist, eliminiert werden. Ikterische und lipämische Proben wurden hergestellt durch Aufstockung von Humanseren mit Bilirubin bzw. Intralipid® analog Glick (Clin Chem 32/3, 470-475 (1986)).

In einem automatisch messenden Analysegerät wie z. B. den Geräten Boehringer Mannheim/Hitachi 704 oder 717 kann die Berechnung des ungestörten Analytwerts so programmiert werden, daß bereits die korrigierten Werte ausgegeben werden und eine nachträgliche rechnerische Korrektur nicht mehr erforderlich ist. Diese Programmierung wird z. B. für Albumin am Boehringer Mannheim/Hitachi 704 Gerät wie folgt durchgeführt:

1. Parameter-Programm: Chemische Parameter:

|  | Test 1 | Test 2 |
|---|---|---|
| Test | (BLALB) | (ALB) |
| Assay Code | 3-15-0 | 3-15-23 |
| Probevolumen ($\mu$l) | 4 | 4 |
| R1-Volumen ($\mu$l) | 350 | 350 |
| R2-Volumen ($\mu$l) | 0 | 350 |
| Wellenlängen | 700-600 | 700-600 |
| Kalibration | K-Faktor | 1-0-0 |
| Std (1) Konz.-Pos (g/l) | 0,00 | 0-1 |
| Std (2) Konz.-Pos (g/l) |  | Sollwert-2 |

2. Monitor: Kalibrationsmonitor (1): Für Test 1 bei S1 Ext. 0 und bei K 100000 eingeben.

3. Der korrigierte Analytwert wird durch "Calculated Test" errechnet (siehe Boehringer Mannheim/Hitachi 704 Manual):

$$\text{Calculated Test} = (\text{Test 2}) - (\text{Test 1}) \cdot F + \text{Konzentration}_{\text{Referenz}}$$

Test 2 ist die Bestimmung der Konzentration des gemessenen unkorrigierten Analytenwerts,

Test 1 ist die Bestimmung der Extinktion des Probenleerwerts,

F ist der für Albumin, Eisen bzw. Total Protein ermittelte Faktor zur Korrektur der Hb-Störung

$\text{Konzentration}_{\text{Referenz}} = F \cdot E1_{\text{Referenz}}$ und wird als Konzentration eingegeben.

Beispiel 1

Ermittlung des Korrekturfaktors für die Bestimmung von Albumin nach der Bromcresolgrün-Methode

Die Bestimmung wurde an einem Boehringer Mannheim/Hitachi 704 Analysegerät mit dem Assay-Code 2-15-23 bei 37 °C durchgeführt. Es wurden folgende Reagenzien verwendet:

Reagenz 1: 75 mmol/l Succinat-Puffer, pH 4,2.
Reagenz 2: 75 mmol/l Succinat-Puffer, pH 4,2; 0,3 mmol/l Bromcresolgrün.

Die Testdurchführung war wie folgt: zu 4 $\mu$l Probe wurden 350 $\mu$l Reagenz 1 und nach Bestimmung des Probenleerwerts 350 $\mu$l Reagenz 2 gegeben. Dann erfolgte nach einer Dauer von 2 min die Bestimmung des Analyten. Zur Messung wurden eine Hauptmeßwellenlänge von 600 nm und eine Nebenmeßwellenlänge von 700 nm verwendet.
Das Ergebnis dieser Bestimmung ist in Tabelle 1 gezeigt. Der Wert für den testspezifischen Korrekturfaktor wurde mit 0,332 ermittelt.

Beispiel 2

Ermittlung des Korrekturfaktors für die Bestimmung von Eisen durch die Ferrozin-Methode

Die Bestimmung wurde am Analysegerät Boehringer Mannheim/Hitachi 717 mit dem Assay-Code 2-24-30 bei 37 °C durchgeführt. Es wurden folgende Reagenzien verwendet:

Reagenz 1: 150 mmol/l Na-Acetat-Puffer, pH 5,0; 4 mmol/l Guanidiniumchlorid; 100 mmol/l Thioharnstoff; Detergenz;

Reagenz 2: 150 mmol/l Ascorbinsäure, 50 mmol/l Ferrozin.

Die Testdurchführung war wie folgt: Zu 20 µl Probe wurden 250 µl Reagenz 1 und nach Bestimmung des Probenleerwerts 50 µl Reagenz 2 gegeben. Die Bestimmung des Analyten erfolgte dann nach einer Dauer von 1 min. Zur Messung wurden eine Hauptmeßwellenlänge von 546 nm und eine Nebenmeßwellenlänge von 700 nm verwendet.
Das Ergebnis des Versuchs ist in Tabelle 2 gezeigt. Es wurde für den testspezifischen Korrekturfaktor ein Wert von 0,290 ermittelt.

Beispiel 3

Ermittlung des Korrekturfaktors für die Bestimmung von Gesamtprotein nach der Biuret-Methode

Die Bestimmung erfolgte an einem Boehringer Mannheim/Hitachi 717 Analysegerät mit dem Assay-Code 2-24-50 bei 37 °C. Es wurden folgende Reagenzien verwendet:

Reagenz 1: 200 mmol/l NaOH; 32 mmol/l K-Na-Tartrat;
Reagenz 2: 200 mmol/l NaOH; 32 mmol/l K-Na-Tartrat; 30,5 mmol/l KJ; 12,15 mmol/l Cu-Sulfat.

Die Testdurchführung war wie folgt: Zu 7 µl Probe wurden 250 µl Reagenz 1 und nach Bestimmung des Probenleerwerts 250 µl Reagenz 2 gegeben. Die Bestimmung des Analyten erfolgte dann nach einer Dauer von 5 min. Zur Messung wurden eine Hauptmeßwellenlänge von 546 nm und eine Nebenmeßwellenlänge von 700 nm verwendet.
Das Ergebnis des Versuchs ist in Tabelle 3 gezeigt. Der Wert für den testspezifischen Korrekturfaktor wurde mit 0,115 bestimmt.

Beispiel 4

Verwendung der Korrekturformel für die Albuminbestimmung

Der in Beispiel 1 ermittelte Korrekturfaktor wurde für die Bestimmung von hämoglobinhaltigen Proben verwendet, die durch Aufstockung mit Blutersatzmittel erhalten wurden.
Die Testdurchführung war wie in Beispiel 1 beschrieben.
Die Formel zur Berechnung der korrigierten Analytkonzentration in der Probe war wie folgt:

$$C'_{Probe} = C_{Probe} - F \cdot E1_{Probe} + F \cdot E1_{Referenz} = C_{Probe} - F \cdot E1_{Probe} + 0{,}3\ g/l.$$

Das Ergebnis dieses Versuchs ist in Tabelle 4 gezeigt. Es ist zu erkennen, daß durch die Korrektur eine Wiederfindungsrate von $100 \pm 1\ \%$ erreicht wurde.

Beispiel 5

Verwendung der Korrekturformel für die Bestimmung von Eisen

Unter Verwendung des in Beispiel 2 ermittelten Korrekturfaktors wurde eine Bestimmung von Eisen nach der Ferrozin-Methode in hämoglobinhaltigen Proben durchgeführt, die durch Aufstockung mit Hämolysat erhalten wurden.
Die Testdurchführung erfolgte wie in Beispiel 2 beschrieben.
Die Formel zur Berechnung des korrigierten Analysenwerts war wie folgt:

$$C'_{Probe} = C_{Probe} - F \cdot E1_{Probe} + F \cdot E1_{Referenz} = C_{Probe} - F \cdot E1_{Probe} + 2{,}9 \; \mu g/dl.$$

Das Ergebnis dieses Versuchs ist in Tabelle 5 dargestellt. Es ist zu erkennen, daß eine hervorragende Wiederfindung von Eisen erreicht wurde, die mit Ausnahme eines einzigen Werts im Bereich von 100 % $\pm$ 2,5 % lag.

Beispiel 6

Verwendung der Korrekturformel für die Bestimmung von Totalprotein

Es wurde unter Verwendung des in Beispiel 3 bestimmten Korrekturfaktors eine Bestimmung von Totalprotein nach der Biuret-Methode für Hämoglobin-haltige Proben durchgeführt, die durch Aufstockung mit Blutersatzmittel erhalten wurden. Die Durchführung des Versuchs erfolgte wie in Beispiel 3 beschrieben.
Die Formel zur Berechnung des korrigierten Analytwerts war wie folgt:

$$C'_{Probe} = C_{Probe} - F \cdot E1_{Probe} + F \cdot E1_{Referenz} = C_{Probe} - F \cdot E1_{Probe} + 0{,}6 \; g/l.$$

Das Ergebnis der Versuche ist in Tabelle 6 dargestellt. Es ist zu erkennen, daß die Wiederfindung für Gesamtprotein in den allermeisten Fällen im Bereich von 100 $\pm$ 1 % lag.

Beispiel 7

Bestimmung von Albumin in ikterischen Proben

Es wurde der in Beispiel 1 ermittelte testspezifische Korrekturfaktor für die Bestimmung von Albumin nach der Bromcresolgrün-Methode bei ikterischen Proben verwendet, die Aufstockung mit Bilirubin erhalten wurden.
Die Testdurchführung erfolgte wie in Beispiel 1 beschrieben. Die Formel zur Berechnung des korrigierten Analytwerts war wie in Beispiel 4 beschrieben.
Das Ergebnis dieses Versuchs ist in Tabelle 7 gezeigt. Es ist zu erkennen, daß die Anwendung der Korrekturformel zu keiner Verschlechterung bei der Wiederfindung führte.

Beispiel 8

Bestimmung von Eisen in lipämischen Proben

Es wurde der in Beispiel 2 ermittelte testspezifische Korrekturfaktor für die Bestimmung von Eisen nach der Ferrozin-Methode bei lipämischen Proben verwendet, die durch Aufstockung mit Intralipid® erhalten wurden.
Die Testdurchführung erfolgte wie in Beispiel 2 beschrieben. Die Formel zur Berechnung des korrigierten Analytwerts war wie in Beispiel 5 angegeben.
Das Ergebnis des Versuchs ist in Tabelle 8 dargestellt. Es ist zu erkennen, daß durch Anwendung der Korrekturformel keine verschlechterte Wiederfindung bei lipämischen Proben erreicht wurde.

| Proben-Nr. | Hb-Gehalt [mg/dl] | E1 Probe [mE] | delta E1 [mE] | C Probe (= gemess.Werte) [g/l] | delta C [g/l] | F Probe [g/l] |
|---|---|---|---|---|---|---|
| 1 (=Referenz) | 0 | 1,0 | - | 32,4 | - | - |
| 2 | 200 | 6,8 | 5,8 | 34,3 | 1,9 | 0,328 |
| 3 | 400 | 12,5 | 11,5 | 36,1 | 3,7 | 0,322 |
| 4 | 600 | 18,1 | 17,1 | 38,0 | 5,6 | 0,328 |
| 5 | 800 | 23,5 | 22,5 | 40,0 | 7,6 | 0,338 |
| 6 | 1000 | 29,0 | 28,0 | 41,8 | 9,4 | 0,336 |
| 7 | 1200 | 34,5 | 33,5 | 43,8 | 11,4 | 0,340 |
| 8 | 1400 | 39,7 | 38,7 | 45,4 | 13,0 | 0,336 |
| 9 | 1600 | 44,9 | 43,9 | 47,0 | 14,6 | 0,333 |
| 10 | 1800 | 50,4 | 49,4 | 48,7 | 16,3 | 0,330 |
| 11 | 2000 | 56,6 | 55,6 | 50,9 | 18,5 | 0,333 |
| | | | | | | F = 0,332 |

Tab. 1

Tab. 2

| Proben-Nr. | Hb-Gehalt [mg/dl] | E1 Probe [mE] | delta E1 [mE] | C Probe (= gemess.Werte) [µg/dl] | delta C [µg/dl] | F Probe [µg/dl] |
|---|---|---|---|---|---|---|
| 1 (=Referenz) | 0 | 10,1 | - | 80,0 | - | - |
| 2 | 100 | 46,8 | 36,7 | 89,3 | 9,3 | 0,253 |
| 3 | 200 | 75,6 | 65,5 | 99,3 | 19,3 | 0,295 |
| 4 | 300 | 114,0 | 103,9 | 110,3 | 30,3 | 0,292 |
| 5 | 400 | 146,2 | 136,1 | 120,3 | 40,3 | 0,296 |
| 6 | 500 | 180,8 | 170,7 | 129,0 | 49,0 | 0,287 |
| 7 | 600 | 214,0 | 203,9 | 140,0 | 60,0 | 0,294 |
| 8 | 700 | 245,4 | 235,3 | 150,3 | 70,3 | 0,299 |
| 9 | 800 | 280,9 | 270,8 | 157,3 | 77,3 | 0,286 |
| 10 | 900 | 314,4 | 304,3 | 166,7 | 86,7 | 0,285 |
| 11 | 1000 | 340,0 | 329,9 | 183,0 | 103,0 | 0,312 |

F = 0,290

Tab. 3

| Proben-Nr. | Hb-Gehalt [mg/dl] | E1 Probe | delta E1 | C Probe (= gemess.Werte) [g/l] | delta C [g/l] | F Probe [g/l] |
|---|---|---|---|---|---|---|
| 1 (=Referenz) | 0 | 5,1 | - | 55,2 | - | - |
| 2 | 200 | 21,7 | 16,6 | 57,0 | 1,8 | 0,108 |
| 3 | 400 | 37,8 | 32,7 | 58,6 | 3,4 | 0,104 |
| 4 | 600 | 53,9 | 48,8 | 61,1 | 5,9 | 0,121 |
| 5 | 800 | 70,9 | 65,8 | 63,4 | 8,2 | 0,125 |
| 6 | 1000 | 86,4 | 81,3 | 64,9 | 9,7 | 0,119 |
| 7 | 1200 | 103,4 | 98,3 | 66,9 | 11,7 | 0,119 |
| 8 | 1400 | 120,1 | 115,0 | 68,5 | 13,3 | 0,116 |
| 9 | 1600 | 135,7 | 130,6 | 70,1 | 14,9 | 0,114 |
| 10 | 1800 | 152,9 | 147,8 | 72,4 | 17,2 | 0,116 |
| 11 | 2000 | 167,8 | 162,7 | 73,5 | 18,3 | 0,112 |

F = 0,115

11

Tab. 4

EP 0 906 569 B1

| Proben-Nr. | Hb-Gehalt [mg/dl] | Gemessene Albumin-Werte | | E1 Probe [mE] | F x E1 Probe [g/l] | Korrigierte Albumin-Werte | |
|---|---|---|---|---|---|---|---|
| | | C Probe [g/l] | Wiederfindung [%] | | | C' Probe [g/l] | Wiederfindung [%] |
| 1 (=Referenz) | 0 | 32,4 | - | 1,0 | F=0,322 g/l<br>0,3 * | 32,4 | - |
| 2 | 200 | 34,3 | 105,9 | 6,8 | 2,3 | 32,3 | 99,7 |
| 3 | 400 | 36,1 | 111,4 | 12,5 | 4,2 | 32,2 | 99,4 |
| 4 | 600 | 38,0 | 117,3 | 18,1 | 6,0 | 32,3 | 99,7 |
| 5 | 800 | 40,0 | 123,5 | 23,5 | 7,8 | 32,5 | 100,3 |
| 6 | 1000 | 41,8 | 129,0 | 29,0 | 9,6 | 32,5 | 100,3 |
| 7 | 1200 | 43,8 | 135,2 | 34,5 | 11,5 | 32,6 | 100,6 |
| 8 | 1400 | 45,4 | 140,1 | 39,7 | 13,2 | 32,5 | 100,3 |
| 9 | 1600 | 47,0 | 145,1 | 44,9 | 14,9 | 32,4 | 100,0 |
| 10 | 1800 | 48,7 | 150,3 | 50,4 | 16,7 | 32,3 | 99,7 |
| 11 | 2000 | 50,9 | 157,1 | 56,6 | 18,8 | 32,4 | 100,0 |
| | | | | | * F x E1 Referenz | | |

| Proben-Nr. | Hb-Gehalt [mg/dl] | Gemessene Eisen-Werte | | E1 Probe [mE] | F x E1 Probe [µg/dl] | Korrigierte Eisen-Werte | |
|---|---|---|---|---|---|---|---|
| | | C Probe [µg/dl] | Wiederfindung [%] | | | C' Probe [µg/dl] | Wiederfindung [%] |
| | | | | | F=0,290 µg/dl | | |
| 1 (=Referenz) | 0 | 80,0 | - | 10,1 | 2,9 * | 80,0 | - |
| 2 | 100 | 89,3 | 111,6 | 46,8 | 13,6 | 78,6 | 98,2 |
| 3 | 200 | 99,3 | 124,1 | 75,6 | 21,9 | 80,3 | 100,4 |
| 4 | 300 | 110,3 | 137,9 | 114,0 | 33,1 | 80,1 | 100,1 |
| 5 | 400 | 120,3 | 150,4 | 146,2 | 42,4 | 80,8 | 101,0 |
| 6 | 500 | 129,0 | 161,2 | 180,8 | 52,4 | 79,5 | 99,4 |
| 7 | 600 | 140,0 | 175,0 | 214,0 | 62,1 | 80,8 | 101,0 |
| 8 | 700 | 150,3 | 187,9 | 245,4 | 71,2 | 82,0 | 102,5 |
| 9 | 800 | 157,3 | 196,6 | 280,9 | 81,5 | 78,7 | 98,4 |
| 10 | 900 | 166,7 | 208,4 | 314,4 | 91,2 | 78,4 | 98,0 |
| 11 | 1000 | 183,0 | 228,8 | 340,0 | 98,6 | 87,3 | 109,1 |
| | | | | | * F x E1 Referenz | | |

Tab. 5

Tab. 6

| Proben-Nr. | Hb-Gehalt [mg/dl] | Gemessene TProtein-Werte | | E1 Probe [mE] | F x E1 Probe [g/l] F=0,115 g/l | Korrigierte TProtein-Werte | |
|---|---|---|---|---|---|---|---|
| | | C Probe [g/l] | Wiederfindung [%] | | | C' Probe [g/l] | Wiederfindung [%] |
| 1 (=Referenz) | 0 | 55,2 | - | 5,1 | 0,6 * | 55,2 | - |
| 2 | 200 | 57,0 | 103,3 | 21,7 | 2,5 | 55,1 | 99,8 |
| 3 | 400 | 58,6 | 106,2 | 37,8 | 4,3 | 54,9 | 99,5 |
| 4 | 600 | 61,1 | 110,7 | 53,9 | 6,2 | 55,5 | 100,5 |
| 5 | 800 | 63,4 | 114,9 | 70,9 | 8,2 | 55,8 | 101,1 |
| 6 | 1000 | 64,9 | 117,6 | 86,4 | 9,9 | 55,6 | 100,7 |
| 7 | 1200 | 66,9 | 121,2 | 103,4 | 11,9 | 55,6 | 100,7 |
| 8 | 1400 | 68,5 | 124,1 | 120,1 | 13,8 | 55,3 | 100,2 |
| 9 | 1600 | 70,1 | 127,0 | 135,7 | 15,6 | 55,1 | 99,8 |
| 10 | 1800 | 72,4 | 131,2 | 152,9 | 17,6 | 55,4 | 100,4 |
| 11 | 2000 | 73,5 | 133,2 | 167,8 | 19,3 | 54,8 | 99,3 |

* F x E1 Referenz

EP 0 906 569 B1

| Proben-Nr. | Bilirubin-Gehalt [mg/dl] | Gemessene Albumin-Werte | | E1 Probe [mE] | F x E1 Probe [g/l] | Korrigierte Albumin-Werte | |
|---|---|---|---|---|---|---|---|
| | | C Probe [g/l] | Wiederfindung [%] | | F=0,332 g/l | C' Probe [g/l] | Wiederfindung [%] |
| 1 (=Referenz) | 0 | 48,3 | - | 0,8 | 0,3 | 48,3 | - |
| 2 | 6 | 47,9 | 99,2 | 0,8 | 0,3 | 47,9 | 99,2 |
| 3 | 13 | 48,0 | 99,4 | 0,9 | 0,3 | 48,0 | 99,4 |
| 4 | 20 | 47,9 | 99,2 | 0,8 | 0,3 | 47,9 | 99,2 |
| 5 | 26 | 48,2 | 99,8 | 0,9 | 0,3 | 48,2 | 99,8 |
| 6 | 33 | 48,2 | 99,8 | 1,4 | 0,5 | 48,0 | 99,4 |
| 7 | 40 | 48,1 | 99,6 | 1,8 | 0,6 | 47,8 | 99,0 |
| 8 | 46 | 48,5 | 100,4 | 1,8 | 0,6 | 48,2 | 99,8 |
| 9 | 53 | 48,8 | 101,0 | 2,0 | 0,7 | 48,4 | 100,2 |
| 10 | 60 | 47,8 | 99,0 | 2,0 | 0,7 | 47,4 | 98,1 |
| 11 | 66 | 48,3 | 100,0 | 2,0 | 0,7 | 47,9 | 99,2 |

Tab. 7

EP 0 906 569 B1

| Proben-Nr. | Intralipid-Gehalt [mg/dl] | Gemessene Eisen-Werte | | E1 Probe [mE] | F x E1 Probe [µg/dl] | Korrigierte Eisen-Werte | |
|---|---|---|---|---|---|---|---|
| | | C Probe [µg/dl] | Wiederfindung [%] | | | C' Probe [µg/dl] | Wiederfindung [%] |
| | | | | | F=0,290 µg/dl | | |
| 1 (=Referenz) | 0 | 82,7 | - | 13,2 | 3,8 | 81,8 | - |
| 2 | 100 | 83,7 | 101,2 | 13,1 | 3,8 | 82,8 | 101,2 |
| 3 | 200 | 84,7 | 102,4 | 13,0 | 3,8 | 83,8 | 102,4 |
| 4 | 300 | 84,3 | 101,9 | 13,2 | 3,8 | 83,4 | 102,0 |
| 5 | 400 | 86,0 | 104,0 | 13,3 | 3,9 | 85,0 | 103,9 |
| 6 | 500 | 81,3 | 98,3 | 13,4 | 3,9 | 80,3 | 98,2 |
| 7 | 600 | 84,3 | 101,9 | 13,6 | 3,9 | 83,3 | 101,8 |
| 8 | 700 | 80,7 | 97,6 | 13,3 | 3,9 | 79,7 | 97,4 |
| 9 | 800 | 86,7 | 104,8 | 13,4 | 3,9 | 85,7 | 104,8 |
| 10 | 900 | 85,3 | 103,1 | 13,4 | 3,9 | 84,3 | 103,1 |
| 11 | 1000 | 82,7 | 100,0 | 13,6 | 3,9 | 81,7 | 99,9 |

Tab. 8

16

**Patentansprüche**

1. Verfahren zur Bestimmung eines Analyten in einer freies Hämoglobin enthaltenden Probe durch optische Messung, wobei man den gemessenen Wert für die Analytkonzentration korrigiert durch die Schritte:

   (a) Messen des Leerwerts der zu analysierenden Probe,
   (b) Messen des Leerwerts einer Hämoglobin freien Referenzprobe,
   (c) Messen des unkorrigierten Werts für die Analytkonzentration und
   (d) Korrigieren des in Schritt (c) erhaltenen Werts durch Korrelation mit den in Schritt (a) und (b) erhaltenen Werten, um den korrigierten Wert für die Analytkonzentration zu erhalten,

   worin der korrigierte Wert für die Analytkonzentration nach folgender Beziehung ermittelt wird:

   $$C'_{Probe} = C_{Probe} - F \cdot E1_{Probe} + F \cdot E1_{Referenz}$$

   wobei

   $C'_{Probe}$ der korrigierte Wert für die Analytkonzentration ist,
   $C_{Probe}$ der unkorrigierte gemessene Wert für die Analytkonzentration in der Probe ist,
   $F$ ein testspezifischer Korrekturfaktor ist,
   $E1_{Probe}$ der gemessene Leerwert in der Probe ist und
   $E1_{Referenz}$ der gemessene Leerwert in der Referenzprobe ist.
   $E1_{Referenz}$ der gemessene Leerwert in der Referenzprobe ist.

2. Verfahren nach Anspruch 1,
   **dadurch gekennzeichnet,**
   **daß** die Bestimmung des Analyten durch eine optische Messung im Bereich von 600-700 nm erfolgt.

3. Verfahren nach einem der Ansprüche 1-2,
   **dadurch gekennzeichnet,**
   **daß** man eine Probe bestimmt, die ein Blutersatzmittel enthält.

4. Verfahren nach einem der Ansprüche 1-3,
   **dadurch gekennzeichnet,**
   **daß** man den Gesamtproteingehalt der Probe bestimmt.

5. Verfahren nach Anspruch 4,
   **dadurch gekennzeichnet,**
   **daß** die Bestimmung des Gesamtproteingehalts nach der Biuret-Methode erfolgt.

6. Verfahren nach einem der Ansprüche 1-3,
   **dadurch gekennzeichnet,**
   **daß** man den Eisengehalt der Probe bestimmt.

7. Verfahren nach Anspruch 6,
   **dadurch gekennzeichnet,**
   **daß** die Bestimmung des Eisengehalts nach der Ferrozin-Methode erfolgt.

8. Verfahren nach einem der Ansprüche 1-3,
   **dadurch gekennzeichnet,**
   **daß** man den Albumingehalt der Probe bestimmt.

9. Verfahren nach Anspruch 8,
   **dadurch gekennzeichnet,**
   **daß** die Bestimmung des Albumingehalts nach der Bromcresolgrün- oder Bromcresolpurpur-Methode erfolgt.

10. Verfahren nach einem der Ansprüche 1-9,

**dadurch gekennzeichnet,**
**daß** man bei der Bestimmung von lipämischen Proben ein Aufhellungsmittel zusetzt.

11. Verfahren nach einem der Ansprüche 1-10,
    **dadurch gekennzeichnet,**
    **daß** die Bestimmung an einer Serum- oder Plasmaprobe durchgeführt wird.

12. Verfahren nach einem der Ansprüche 1-11,
    **dadurch gekennzeichnet,**
    **daß** man als Referenzprobe Serum- oder Plasmaproben von klinisch gesunden Probanden verwendet.

13. Verfahren nach einem der Ansprüche 1-12,
    **dadurch gekennzeichnet,**
    **daß** man das Verfahren in einem Analyseautomaten durchführt.

14. Verfahren nach Anspruch 13,
    **dadurch gekennzeichnet,**
    **daß** der Analyseautomat so programmiert wird, daß bereits der korrigierte Wert für die Analytkonzentration ausgegeben wird.

15. Verfahren nach Anspruch 1,
    **dadurch gekennzeichnet,**
    **daß** die Bestimmung des testspezifischen Korrekturfaktors F die Schritte umfaßt:

    (a) Bereitstellen einer Serie von mindestens 3 Proben mit gleichem Analytgehalt, wobei mindestens eine der Proben kein Hämoglobin enthält und mindestens 2 der Proben unterschiedliche Konzentrationen an freiem Hämoglobin enthalten,

    (b) Messen des Leerwerts von jeder Probe, wobei der durch Anwesenheit von Hämoglobin verursachte Anstieg des Probenleerwerts gegenüber der Hämoglobin-freien Probe bestimmt wird,

    (c) Messen der unkorrigierten Analytkonzentration in jeder Probe, wobei die durch Anwesenheit von Hämoglobin verursachte Meßwertverfälschung gegenüber der Hämoglobin-freien Referenzprobe bestimmt wird, und

    (d) Korrelation der in Schritt (c) bestimmten Meßwertverfälschung mit dem in Schritt (b) bestimmten Anstieg des Probenleerwerts, um den testspezifischen Korrekturfaktor zu erhalten.

16. Verfahren nach Anspruch 15,
    **dadurch gekennzeichnet,**
    **daß** man eine Serie von Proben bereitstellt, wobei mindestens 5 der Proben unterschiedliche Konzentrationen an freien Hämoglobinen enthalten.

17. Verfahren nach Anspruch 15 oder 16,
    **dadurch gekennzeichnet,**
    **daß** man eine Serie von Proben bereitstellt, deren Konzentration von 0 mg/dl bis mindestens 1000 mg/dl freies Hämoglobin variiert.

18. Verfahren nach einem der Ansprüche 15-17,
    **dadurch gekennzeichnet,**
    **daß** ein probenspezifischer Korrekturfaktor F' für eine freies Hämoglobin enthaltende Probe nach folgender Beziehung ermittelt wird:

$$F' = \Delta C : \Delta E1$$

wobei:

Δ C der für jeweils eine Probe durch die Anwesenheit von freiem Hämoglobin verursachte Betrag der Meßwertverfälschung gegenüber der Referenzprobe ist und

Δ E1 der für jeweils eine Probe durch die Anwesenheit von freiem Hämoglobin verursachte Anstieg des Probenleerwerts gegenüber der Referenzprobe ist,

und **daß** der testspezifische Korrekturfaktor F durch Bildung des Mittelwerts aus den für die jeweiligen Einzelproben bestimmten Korrekturfaktoren F' ermittelt wird.

**Claims**

1.  Method for the determination of an analyte in a sample containing free haemoglobin by optical measurement wherein the measured value for the analyte concentration is corrected by the steps:

    (a) measuring the blank value of the sample to be analysed,
    (b) measuring the blank value of a haemoglobin-free reference sample,
    (c) measuring the uncorrected value for the analyte concentration and
    (d) correcting the value obtained in step (c) by correlation with the values obtained in steps (a) and (b) in order to obtain the corrected value for the analyte concentration,

    wherein the corrected value for the analyte concentration is calculated according to the following relation:

    $$C'_{sample} = C_{sample} - F \cdot E1_{sample} + F \cdot E1_{reference}$$

    in which

    $C'_{sample}$ is the corrected value for the analyte concentration,
    $C_{sample}$ is the uncorrected measured value for the analyte concentration in the sample,
    F is a test-specific correction factor,
    $E1_{sample}$ is the measured blank value in the sample and
    $E1_{reference}$ is the measured value in the reference sample.

2.  Method as claimed in claim 1,
    **wherein**
    the determination of the analyte is carried out by an optical measurement in the range of 600-700 nm.

3.  Method as claimed in one of the claims 1-2,
    **wherein**
    a sample is determined which contains a blood substitute.

4.  Method as claimed in one of the claims 1-3,
    **wherein**
    the total protein content of the sample is determined.

5.  Method as claimed in claim 4,
    **wherein**
    the determination of the total protein content is carried out according to the Biuret method.

6.  Method as claimed in one of the claims 1-3,
    **wherein**
    the iron content of the sample is determined.

7.  Method as claimed in claim 6,
    **wherein**
    the iron content is determined according to the ferrozine method.

**8.** Method as claimed in one of the claims 1-3,
**wherein**
the albumin content of the sample is determined.

**9.** Method as claimed in claim 8,
wherein
the albumin content is determined according to the bromocresol-green or the bromocresol-purple method.

**10.** Method as claimed in one of the claims 1-9,
**wherein**
in the determination of lipaemic samples a clearing agent is added.

**11.** Method as claimed in one of the claims 1-10,
**wherein**
the determination is carried out on a serum or plasma sample.

**12.** Method as claimed in one of the claims 1-11,
**wherein**
serum or plasma samples of clinically healthy test persons are used as a reference sample.

**13.** Method as claimed in one of the claims 1-12,
**wherein**
the method is carried out in an automated analyzer.

**14.** Method as claimed in claim 13,
**wherein**
the automated analyzer is programmed in such a way that the corrected value for the analyte concentration is already printed out.

**15.** Method as claimed in claim 1,
**wherein**
the determination of the test-specific correction factor F comprises the steps:

(a) Preparing a series of at least three samples with the same content of analyte of which at least one of the samples contains no haemoglobin and at least two of the samples contain different concentrations of free haemoglobin,

(b) measuring the blank value of each sample to determine the increase of the sample blank value caused by the presence of haemoglobin compared to the haemoglobin-free sample,

(c) measuring the uncorrected analyte concentration in each sample to determine the falsification of the measured value caused by the presence of haemoglobin compared to the haemoglobin-free reference sample and

(d) the falsification of the measured value determined in step (c) is correlated with the increase of the sample blank value determined in step (b) in order to obtain the test-specific correction factor.

**16.** Method as claimed in claim 15,
**wherein**
a series of samples is prepared of which at least 5 of the samples contain different concentrations of free haemoglobin.

**17.** Method as claimed in claim 15 or 16,
**wherein**
a series of samples is prepared the concentration of which varies from 0 mg/dl to at least 1000 mg/dl of free haemoglobin.

**18.** Method as claimed in one of the claims 15-17,
**wherein**

a sample-specific correction factor F' is determined for a sample containing free haemoglobin according to the following relationship:

$$F' = \Delta C : \Delta E1$$

in which:

$\Delta C$ is the amount of the falsification of the measured value compared to the reference sample caused by the presence of free haemoglobin for an individual sample and

$\Delta E1$ is the increase of the sample blank value compared to the reference sample caused by the presence of free haemoglobin for an individual sample,

and the test-specific correction factor F is determined by calculating the mean of the correction factors F' determined for the respective individual samples.


**Revendications**

1. Procédé pour la détermination d'un analyte dans un échantillon contenant de l'hémoglobine libre par la mesure optique, moyennant quoi on corrige la valeur mesurée pour la concentration d'analyte par les étapes consistant à :

   (a) mesurer la valeur vide de l'échantillon à analyser,
   (b) mesurer la valeur vide d'un échantillon de référence exempt d'hémoglobine,
   (c) mesurer la valeur non corrigée pour la concentration d'analyte et
   (d) corriger la valeur contenue à l'étape (c) par corrélation avec les valeurs contenues à l'étape (a) et (b) pour obtenir la valeur corrigée pour la concentration d'analyte,

   la valeur corrigée pour la concentration d'analyte étant déterminée selon la relation suivante :

   $C'_{\text{échantillon}} = C_{\text{échantillon}} - F \cdot E1_{\text{échantillon}} + F \cdot E1_{\text{référence}}$
   $C'_{\text{échantillon}}$ étant la valeur corrigée pour la concentration d'analyte,
   $C_{\text{échantillon}}$ étant la valeur mesurée non corrigée pour la concentration d'analyte dans l'échantillon,
   F étant un facteur de correction spécifique au test,
   $E1_{\text{échantillon}}$ étant la valeur vide mesurée de l'échantillon et
   $E1_{\text{référence}}$ étant la valeur vide mesurée dans l'échantillon de référence.

2. Procédé selon la revendication 1,
   **caractérisé en ce que**
   la détermination de l'analyte s'effectue par une mesure optique dans une plage de 600-700nm.

3. Procédé selon l'une des revendications 1-2,
   **caractérisé en ce que**
   l'on détermine un échantillon qui contient un succédané du sang.

4. Procédé selon l'une des revendications 1-3,
   **caractérisé en ce que**
   l'on détermine la teneur totale en protéines de l'échantillon.

5. Procédé selon la revendication 4,
   **caractérisé en ce que**
   la détermination de la teneur totale en protéines s'effectue selon la méthode biuret.

6. Procédé selon l'une des revendications 1-3,
   **caractérisé en ce que**
   l'on détermine le teneur en fer de l'échantillon.

**7.** Procédé selon la revendication 6,
**caractérisé en ce que**
la détermination de la teneur en fer s'effectue selon la méthode ferrozin.

**8.** Procédé selon l'une des revendications 1-3,
**caractérisé en ce que**
l'on détermine la teneur en albumine de l'échantillon.

**9.** Procédé selon la revendication 8,
**caractérisé en ce que**
la détermination de la teneur en albumine s'effectue selon la méthode du vert de bromocrésol ou violet de bromocrésol.

**10.** Procédé selon une des revendications 1-9,
**caractérisé en ce que**
lors de la détermination des échantillons lipémiques, on ajoute un agent d'éclaicissage.

**11.** Procédé selon l'une des revendications 1-10,
**caractérisé en ce que**,
la détermination est effectuée sur un échantillon de sérum ou de plasma.

**12.** Procédé selon l'une des revendications 1-11,
**caractérisé en ce que**
comme échantillon de référence, on utilise des échantillons de sérum ou de plasma de sujets d'expérimentation cliniquement sains.

**13.** Procédé selon l'une des revendications 1-12,
**caractérisé en ce que**
l'on exécute le procédé dans un automate d'analyse.

**14.** Procédé selon la revendication 13,
**caractérisé en ce que**
l'automate d'analyse est programmé de telle sorte que la valeur corrigée pour la concentration d'analyte est déjà émise.

**15.** Procédé selon la revendication 1,
**caractérisé en ce que**
la détermination du facteur de correction F spécifique au test comprend les étapes consistant à :

(a) mettre à disposition une série d'au moins 3 échantillons avec la même teneur en analyte, au moins l'un des échantillons ne contenant pas d'hémoglobine et au moins 2 des échantillons contenant des concentrations différentes d'hémoglobine libre.

(b) mesurer la valeur vide de chaque échantillon, l'augmentation de la valeur vide d'échantillon provoquée par la présence d'hémoglobine étant déterminée par rapport à l'échantillon exempt d'hémoglobine,

(c) mesure de la concentration d'analyte corrigée dans chaque échantillon, la déformation de la valeur mesurée provoquée par la présence d'hémoglobine étant déterminée par rapport à l'échantillon de référence exempt d'hémoglobine, et

(d) corréler la déformation de la valeur mesurée déterminée à l'étape (b) avec l'augmentation de la valeur de vide d'échantillon déterminée à l'étape (b) pour obtenir le facteur de correction spécifique au test.

**16.** Procédé selon la revendication 15,
**caractérisé en ce que**
l'on met à disposition une série d'échantillons, au moins 5 des échantillons contenant des concentrations différentes d'hémoglobines libres.

17. Procédé selon la revendication 15 ou 16,
    **caractérisé en ce que**
    l'on met à disposition une série d'échantillons dont la concentration varie de 0 mg/dl jusqu'à au moins 1000 mg/dl d'hémoglobine libre.

18. Procédé selon l'une des revendications 15-17,
    **caractérisé en ce que**
    l'on calcule un facteur de correction F' spécifique à l'échantillon pour un échantillon contenant de l'hémoglobine libre selon la relation suivante :

$$F'=\Delta C : \Delta E1$$

$\Delta C$ étant la somme de la déformation de valeur mesurée provoquée par la présence d'hémoglobine libre par rapport à l'échantillon de référence et

$\Delta E1$ étant l'augmentation de la valeur de vide d'échantillon par rapport à l'échantillon de référence provoquée par la présence d'hémoglobine libre,

et **en ce que** le facteur de correction F spécifique au test est calculé par formation de la valeur moyenne à partir des facteurs de correction F' déterminés pour les échantillons individuels respectifs.